(19) Europäisches Patentamt

European Patent Office

Office européen des brevets

(11) EP 1 587 758 B1

(12) **EUROPEAN PATENT SPECIFICATION**

(45) Date of publication and mention
of the grant of the patent:
**12.11.2008 Bulletin 2008/46**

(51) Int Cl.:
*C01G 23/07* (2006.01)     *C01G 23/00* (2006.01)
*A61K 8/00* (2006.01)      *C08K 3/36* (2006.01)
*C09C 1/36* (2006.01)      *H01G 9/20* (2006.01)

(21) Application number: **03768325.7**

(22) Date of filing: **26.12.2003**

(86) International application number:
**PCT/JP2003/016964**

(87) International publication number:
**WO 2004/060808 (22.07.2004 Gazette 2004/30)**

(54) **PRODUCTION PROCESS OF TITANIA-SILICA MIXED CRYSTAL PARTICLES HAVING A HIGH BULK DENSITY, TITANIA-SILICA MIXED CRYSTAL PARTICLES OBTAINED BY THE PROCESS AND USES THEREOF**

HERSTELLUNGSPROZESS FÜR KRISTALLINISCHE TITANDIOXID-SILIKA-MISCHPARTIKEL MIT HOHER SCHÜTTDICHTE, HIERDURCH ERHALTENE KRISTALLINISCHE TITANDIOXID-SILIKA-MISCHPARTIKEL SOWIE DEREN VERWENDUNG

PROCEDE DE PRODUCTION DE PARTICULES MIXTES DE CRISTAUX D'OXYDE DE TITANE/SILICE A FORTE DENSITE APPARENTE, PARTICULES AINSI OBTENUES, ET LEURS UTILISATIONS

(84) Designated Contracting States:
**AT BE BG CH CY CZ DE DK EE ES FI FR GB GR HU IE IT LI LU MC NL PT RO SE SI SK TR**

(30) Priority: **27.12.2002 JP 2002381800**

(43) Date of publication of application:
**26.10.2005 Bulletin 2005/43**

(73) Proprietor: **Showa Denko K.K.**
**Tokyo 105-8518 (JP)**

(72) Inventors:
• **KOGOI, Hisao**
**Toyama-shi, Toyama 931-8577 (JP)**

• **TANAKA, Jun**
**Corporate R & D Center**
**Chiba-shi, Chiba 267-0056 (JP)**

(74) Representative: **Strehl Schübel-Hopf & Partner**
**Maximilianstrasse 54**
**80538 München (DE)**

(56) References cited:
US-A- 5 762 914        US-A- 6 022 404
US-A1- 2002 114 761

• DATABASE WPI Section Ch, Week 197602 Derwent Publications Ltd., London, GB; Class A60, AN 1976-02758X XP002275894 & JP 50 115190 A (MITSUBISHI CHEM IND LTD) , 9 September 1975 (1975-09-09)

## Description

Field of the Invention

The present invention relates to a process for producing titania-silica mixed crystal particles comprising titanium oxide as the main component and silica as a subsidiary component, which is a powder having a large specific surface area and, nevertheless, does not grow to have a steric structure constituted by connected primary or secondary particles because of a low aggregation degree of primary particles and therefore, can be easily dispersed or suspended in a non-aqueous solvent, an aqueous solvent, an organic polymer composition including resin, or a silicon polymer composition. The present invention also relates to titanium-silica mixed crystal particles obtained by the production process and uses thereof.

Background Art

In recent years, the industrial applications of ultrafine particulate titanium oxide are largely expanding and studies are being made on applications over a wide range, for example, a dielectric raw material, a filler for a resin film or the like, a bulking agent for organic polymer compositions or silicon polymer compositions, an ultraviolet-shielding material, a silicon rubber additive and a coating agent intended to have a photocatalytic ability.

On the other hand, a titania-silica mixed crystal based on titanium oxide has an improved dispersibility in resin, solvent, oil and fat, silicon polymer or the like as compared with titanium oxide. Furthermore, the titania-silica mixed crystal has heat resistance to undergoing less reduction in specific surface area even at high temperatures and, therefore, development of uses over a wider range than for titanium oxide are expected.

As a production method for composite particles containing titanium oxide, a method of producing silica-titania composite particles by reacting a mixed vapor of a silicon halide and a titanium halide with an oxidizing gas containing oxygen at a temperature of 900°C or more is known (see, JP-A-50-115190 (the term "JP-A" as used herein means an "unexamined published Japanese patent application"). In this method, the mixed raw material vapor is reacted at a high temperature of 900°C or more without performing preheating and the composite powder produced has a structure such that crystalline $TiO_2$ particles are always deposited on a surface.

Also, Japanese Patent No. 2,503,370 (European Patent No. 595,078) discloses that a titania-silica mixed crystal comprising titanium oxide-aluminum oxide-silicon oxide can be produced by flame hydrolysis using a chloride as a raw material (the reaction temperature is from 1,000 to 3,000°C). As flame hydrolysis is employed, the product is an alumina-titania mixed crystal or a titania-silica mixed crystal.

Furthermore, JP-T-9-511985 (the term "JP-T" as used herein means a "published Japanese translation of PCT patent application") discloses a method of obtaining anatase-free titanium oxide by adding a silicon halide to a plug flow reactor. Other than these, U.K. Patent No. 689,123 and U.S. Patent No. 3,219,468 are known.

As for the liquid phase method, JP-A-2001-139331 discloses a method of obtaining a titania-silica mixed crystal by using a composite coprecipitate of titanium and silicon.

The titanium oxide or titania-silica mixed crystal can express its function or can be added more effectively when formed into fine particles. For example, in cosmetics, transparency increases by formation into a fine particles and, therefore, unacceptable whiteness can be prevented on application to skin. In use in resin films, coating materials or the like, transparency increases in addition to ultraviolet-shielding ability, photocatalytic ability and the like and therefore, excellent design property is imparted to dramatically expand the use in industry.

In the case of use as a photocatalyst, a powder as a photocatalyst must be fixed on the surface of a structure. This fixing is usually performed by suspending the powder in a coating material or the like and applying it to the surface of a structure. The powder having a photocatalytic function more readily expresses its function as the surface area is larger, because contact with the environment increases. That is, fine particles are preferred. Ultrafine particles have not been clearly defined but, in general, fine particles having a primary particle size of about 0.1 μm or less are called ultrafine particles.

By taking account of uses of the above-described titania-silica mixed crystal particles, an object of the present invention is to provide titania-silica mixed crystal fine particles with low aggregation degree, and a production process and uses thereof.

Finer particles are more difficult to suspend in a base material such as oil and fat, wax or resin. This is considered to be because, as the particles are finer, a steric structure constituted by long connected or massively aggregated primary particles is more readily developed to include the base material in the structure. In other words, particles having such a structure are, even if suspended, very difficult to uniformly disperse and, in an extreme case, powder particles aggregate to cause gelation or solidification. In order to prevent this phenomenon, a technique of hydrophobing the surface with silicone or the like is used in some cases. However, as the particles become finer, a surface treatment of higher level is required and the working efficiency decreases.

The particle structure is described below. In many cases, particles exist as a secondary particle resulting from aggregation of a plurality of primary particles into lumped or chained particles. Also, secondary particles are often connected, by intermolecular force or the like, to take a steric structure. These secondary particles and steric structures are sometimes collectively called aggregated particles. When primary particles do not form a lump, that is, primary particles are not aggregated and exist as individual transfer units, this is called monodispersion. However, this state is very difficult to attain with particles of 1 μm or less.

Usually, even if the primary particles are fine particles, the secondary particle is not a fine particle. That is, as the primary particles become finer, the aggregation degree tends to be higher. The particles having such a high aggregation degree are not only inferior in transparency but also in the handling of the powder, because a solvent or the like is readily included in spaces between primary particles or in voids of the steric structure. Therefore, the particles have a problem in its practical use.

Accordingly, in the formation of fine particles, it is very important to reduce the particle size of the primary particles and also to suppress the formation of secondary particles and steric structures resulting from connection of these particles. However, the above-described patent publications all are silent on the control of such an aggregated structure. A method of controlling the aggregated structure is described in JP-A-10-194741. According to this method, titanium dioxide particles are consolidated by a pressure roll to a bulk density of 0.8 g/cm$^3$ or more. This is intended to attain efficiency of transportation but, from the standpoint of more successfully dispersing the particles on use, the powder is excessively consolidated.

For solving these problems regarding the aggregated structure peculiar to fine particle, various proposals have been made.

Summary of the Invention

The present inventors have taken note of the method for controlling primary particles in the gas phase process and also of the steric or aggregated structure of particles in the powder produced by the gas phase process and achieved the present invention. More specifically, it has been found that a method of controlling the primary particle size by the reaction system for the production of particles and then utilizing mechanical shearing is suitable for the control of steric or aggregated structure of a powder produced by a gas phase process and this results in production of a powder having a low aggregation degree. The present invention has been accomplished based on this finding. It has also been found that the bulk density is effective as an index for steric structure.

As a result of extensive investigations made by taking account of conventional techniques, it has been found that when a mixed gas of titanium halide and silicon halide (hereinafter, generically called a "mixed gas") and an oxidizing gas are each preheated at 600°C or more and then reacted and the obtained powder is heated at 500 to 800°C to decrease the HCl concentration in the powder to 1 mass% or less and then subjected to a treatment for dissociating the aggregated or steric structure of particles, preferably a stirring treatment in a vessel having rotary blades, a titania-silica mixed crystal particle having a bulk density of 0.15 to 0.8 g/cm$^3$ and a BET

specific surface area of 10 to 200 m$^2$/g, preferably a bulk density of 0.2 to 0.6 g/cm$^3$ and a BET specific surface area of 20 to 100 m$^2$/g, more preferably a bulk density of 0.2 to 0.5 g/cm$^3$ and a BET specific surface area of 30 to 70 m$^2$/g, can be obtained. By this finding, the above-described object can be attained. That is, the present invention provides the followings.

(1) A process for producing a titania-silica mixed crystal particle having a high bulk density and comprising titanium oxide as the main component and silicon oxide as a subsidiary component, the process comprising decomposing gaseous titanium halide and gaseous silicon halide each heated at 600°C or more in the presence of oxygen or water vapor heated at 600°C or more, the residence time of the gaseous titanium halide and gaseous silicon halide at a high temperature of 600°C or more being 1 second or less, to obtain a powder comprising titanium oxide and silicon oxide, heating the obtained powder at 500 to 800°C to decrease the concentration of raw material-originated hydrogen halide in the powder to 1 mass% or less, and then subjecting the powder to a treatment of dissociating the aggregated or steric structure.

(2) The process as described in (1) above wherein, in the step of said decomposition, the gaseous metal halide and an oxidizing gas are introduced into a reactor at a flow rate of 30 m/sec or more.

(3) The process as described in (2) above, wherein the gaseous metal halide and the oxidizing gas have an average flow rate of 5 m/sec or more in the reactor.

(4) The process as described in (2) above, wherein the gaseous metal halide and the oxidizing gas have a residence time at a temperature of 600°C or more in the reactor is 1 second or less.

(5) The process for producing a titania-silica mixed crystal particle having a high bulk density as described in any one of (1) to (4) above, wherein the treatment of dissociating the aggregated or steric structure is a stirring treatment of charging the powder into a vessel having a plurality of rotary blades differing in the shape and rotating the rotary blades at a peripheral speed of 4 to 60 m/s.

(6) The process according to (5) above, wherein the dissociating treatment of the powder is conducted in a Henschel mixer.

(7) Titania-silica mixed crystal particles obtainable by the production process described in any one of (1) to (6) above, which have a BET specific surface area of 10 to 200 m$^2$/g and a bulk density of 0.15 g/cm$^3$ to less than 0.8 g/cm$^3$.

(8) Titania-silica mixed crystal particles as described in (7) above, which have a BET specific surface area of 20 to 100 m$^2$/g and a bulk density of 0.2 g/cm$^3$ to less than 0.6 g/cm$^3$.

(9) Titania-silica mixed crystal particles as described in any one of (7) and (8) above, which have a BET specific surface area of 30 to 70 m$^2$/g and a bulk density or 0.2 g/cm$^3$ to less than 0.5 g/cm$^3$.

(10) The titania-silica mixed crystal particleras described in any one of (7) to (9) above, wherein SiO$_2$, is contained in an amount of 0.1 mass% to less than 50 mass%.

(11) The titania-silica mixed crystal particles as described in (10) above, wherein SiO$_2$ is contained in an amount of 10 to 40 mass%.

(12) The titania-silica mixed crystal particles as described in (10) above, wherein SiO$_2$ is contained in an amount of 15 to 30 mass%.

(13) The titania-silica mixed crystal particles as described in any one of (7) to (12) above, wherein the oil absorption amount is less than 1 ml/g as measured by the oil absorption measuring method of JIS K 5101 using squalane in place of linseed oil.

(14) A cosmetic material comprising the titania-silica mixed crystal particle described in any one of (7) to (13) above.

(15) The cosmetic material as described in (14) above, further comprising an additive selected from the group consisting of oils, whitening agents, moisturizers, anti-aging agents, emollients, essences, antiinflammatorys, antioxidants, surfactants, chelating agents, antibiotics, antiseptics, amino acids, sugars, organic acids, alcohols, esters, fats and oils, hydrocarbons, ultraviolet inhibitors and inorganic powders.

(16) An organic polymer composition comprising an organic polymer and the titania-silica mixed crystal particle described in any one of (7) to (13) above, the titania-silica mixed crystal particles being contained in an amount of 0.01 to 80 mass% based on the total mass of the composition.

(17) The organic polymer composition as described in (16) above, wherein the organic polymer of the organic polymer composition is at least one resin selected from the group consisting of synthetic thermoplastic resins, synthetic thermosetting resins and natural resins.

(18) A silicon polymer composition comprising a silicon polymer and the titania-silica mixed crystal particles described in any one of (7) to (13) above, the titania-silica mixed crystal particles being contained in an amount of 0.01 to 90 mass% based on the total mass of the composition.

(19) The organic polymer composition or silicon polymer composition as described in any one of (15) to (18) above, wherein the organic polymer composition or silicon polymer composition is a compound.

(20) The organic polymer composition or silicon polymer composition as described in any one of (15) to (18) above, wherein the organic polymer composition or silicon polymer composition is a masterbatch.

(21) A molded article obtained by molding the organic polymer composition or silicon polymer composition described in any one of (15) to (20) above.

(22) The molded article described in (21) above wherein the molded article is one selected from the group consisting of fiber film and plastic molded article.

(23) A slurry comprising the titania-silica mixed crystal particle described in any one of (7) to (13) above.

(24) A dye-sensitized solar cell comprising the titania-silica mixed crystal particle described in any one of (7) to (13) above in the structure.

(25) A coating agent comprising the titania-silica mixed crystal particle described in any one of (7) to (13) above in water or an organic solvent and optionally a binder.

(26) A coating material comprising the titania-silica mixed crystal particle described in any one of (7) to (13) above in water or an organic solvent and optionally a binder.

(27) A structure having on the surface thereof the titania-silica mixed crystal particle described in any one of (7) to (13) above.

(28) The structure as described in (27) above, which is selected from the group consisting of building materials, machines, vehicles, glass products, home electric appliances, agricultural materials, electronic equipment, tools, tableware, bath furnishings, toilet goods, furniture, clothing, cloth products, fibers, leather products, paper products, sporting goods, bedding, containers, spectacles, billboards, piping, wiring, metal fittings, hygiene materials, automobile equipment, outdoor products such as tents, stockings, socks, gloves and masks.

(29) A photocatalyst which is the titania-silica mixed crystal particle described in any one of (7) to (13) above.

Brief Description of the Drawings

Fig 1 is a schematic view showing one example of a reaction apparatus where the production process of the present invention is practiced.

Fig. 2 is a schematic view showing one example of a volume reducer suitable for the treatment of dissociating the aggregated or steric structure of powder.

Best Modes of Carrying out the Preferred Embodiment of the Invention

The present invention is described in detail below.

According to the present invention, it has been found that, in a gas phase production process of producing titania-silica mixed crystal particles by oxidizing titanium halide and silicon halide with an oxidizing gas at a high temperature, when the mixed gas and the oxidizing gas are reacted, after preheating each gas at 600°C or more, and then passed through a high purity treatment and a volume reduction treatment (aggregating treatment), ultrafine titania-silica mixed crystal particles having a bulk density of 0.15 to 0.8 $g/cm^3$ and a BET specific surface area of 10 to 200 $m^2/g$, preferably a bulk density of 0.2 to 0.6 $g/cm^3$ and a BET specific surface area of 20 to 100 $m^2/g$, more preferably a bulk density of 0.2 to 0.5 $g/cm^3$ and a BET specific surface area of 30 to 70 $m^2/g$, can be obtained. Furthermore, ultrafine titania-silica mixed crystal particles containing silica in a proportion of 0.1 mass% to less than 50 mass%, preferably from 10 to 40 mass%, more preferably from 15 to 30 mass%, can be produced.

In the above-described production process of ultrafine titania-silica mixed crystal particles, the mixed gas is a gas of metal halides selected from the group consisting of chloride, bromide and iodide of titanium and of silicon. As for the form of supplying the metal halide gas to a reaction tube, a gas obtained by individually gasifying the metal halides and then mixing the resulting gases is preferably used. The oxidizing gas is oxygen, water vapor or a mixed gas containing oxygen or water vapor.

The chloride, bromide or iodide of titanium and silicon for use in the present invention is not limited and any metal halide may be used as long as it can produce the metal halide gas when preheated at least at 600°C or more. Preferred examples thereof include $TiCl_2$, $TiCl_3$, $TiCl_4$, $TiBr_3$, $TiBr_4$, $SiCl_4$, $Si_2Cl_6$, $Si_3Cl_8$, $Si_4Cl_{10}$, $Si_5Cl_{12}$, $Si_{10}Cl_{12}$, $SiBr_4$, $Si_2Br_6$, $Si_3Br_8$, $Si_4Br_{10}$, $SiI_4$, $Si_2I_6$, $SiCl_2I_2$, $SiClI_3$, $SiBr_3I$, $SiHI_3$, $SiCl_3I$, $SiH_3Br$, $SiH_2Br_2$, $SiHBr_3$, $SiCl_3Br$, $SiCl_2Br_2$ and $SiClBr_3$.

In the present invention, the mixed metal halide gas and the oxidizing gas each must be preheated at least at 600°C or more, preferably 800°C or more, before the reaction. If the preheating temperature of the mixed metal halide gas and the oxidizing gas is less than 600°C, an ultrafine particle is difficult to obtain due to low reactivity and the raw material-originated halogen portion in the product cannot be satisfactorily removed even by a dehalogenation reaction. When the temperature at the introduction of gas into the reaction tube is 600°C or more, the reaction is completed at the same time with the mixing and, as a result, generation of uniform nuclei is promoted and the primary particle becomes small.

In the present invention, the flow rate at the introduction of the mixed metal halide gas and the oxidizing gas to the reaction tube is 30 m/sec or more, preferably 50 m/sec or more. This is because when the flow rate is elevated, the Reynolds number is increased and the mixing of the gases is accelerated. For obtaining a powder having a low aggregation degree, it is very important that the gas introduced into the reaction tube is swiftly and completely oxidized and the residence time at a high temperature of 600°C or more is 1 second or less. The swift oxidation proceeds by swiftly performing thorough mixing and therefore, the gas fluid state in the reaction tube is preferably a turbulent flow.

In the present invention, the gases supplied into the reaction tube preferably flow at a high flow rate inside the reaction tube for completely performing the mixing of gases, particularly preferably at an average flow rate of 5 m/sec or more. When the average flow rate of gas inside the reaction tube is 5 m/sec or more, thorough mixing can be performed inside the reaction tube.

With respect to the inlet nozzle for introducing the raw material gas into the reaction tube, a nozzle of giving a coaxial parallel flow, an oblique flow, a cross flow or the like is employed but the present invention is not limited thereto. In general, the coaxial parallel flow nozzle is preferably used, because the structure is simple, though this nozzle is inferior in the mixing degree to the nozzle of giving an oblique flow or a cross flow. For example, in the case of a coaxial parallel flow nozzle, the gas containing chloride is introduced into the inner tube and the oxidizing gas is introduced into the outer tube.

The reaction in the reaction tube is an exothermic reaction. Although heat is lost by heat transfer from the reaction apparatus, unless the produced fine particles are rapidly cooled after the reaction, sintering of the particles proceeds to cause the growth of particle size. In the present invention, the particles are preferably controlled to reside in the reaction tube at a high temperature exceeding 600°C for 1 second or less by adjusting the gas flow rate or the size of reaction tube and then are rapidly cooled, whereby aggregated particles are hardly formed.

For rapidly cooling the particle after the reaction, for example, a method of introducing a large amount of cooling air or gas such as nitrogen into the mixture after the reaction, or a method of spraying water is employed.

As the raw material mixed metal halide gas, the above-described mixed metal halide gas may be used in 100 vol% but is preferably diluted with an inert gas to a concentration of 10 vol% to less than 100 vol%, more preferably from 20 vol% to less than 100 vol%. By using a gas having a mixed metal halide gas concentration (a total concentration of metal halide gases) of 10 vol% or more as the raw material, generation of uniform nuclei increases and the reactivity is also elevated. As the inert gas, a gas which does not react with the mixed metal halide and is not oxidized should be selected. Specific preferred examples of the diluting gas include nitrogen and argon.

In the thus-obtained powder, by-products such as hydrogen chloride, hydrogen bromide and hydrogen iodide are adsorbed on the particle surface. To increase the purity of product, these adsorbed by-products are preferably removed by heating. Specifically, a method of heating the powder in a rotary kiln is employed. In order to attain high purity while suppressing the growth of particles due to heating, the heating temperature is usually set to less than 500°C and the residence time in the kiln is prolonged according to the purity demanded. However, the titania-silica mixed crystal ultrafine particles produced by the process of the present invention grow less at a high temperature, that is, this particle has excellent heat resistance and therefore, the heat treatment temperature is set to from 500 to 800°C in the present invention.

This high temperature treatment is considered to affect the aggregation degree of particles or the steric structure and is regarded as an important pre-stage step for obtaining the particle of the present invention. The steps so far are called a particle synthesis process.

The particles produced by the synthesis process of the present invention are particles characterized in that the primary particle size is small and the aggregated or steric structure is readily dissociated (reduced) by a simple and easy volume reduction step. Accordingly, by subjecting the particles produced by the above-described particle synthesis process to a volume reduction treatment of appropriately destroying the steric or aggregated structure composed of chained primary or secondary particles, titania-silica mixed crystal particles having a desired high bulk density can be obtained. In the case of the synthesized particles, the objective volume reduction treatment, that is, the treatment of dissociating the aggregated or steric structure does not require any particular strong shearing or consolidation force and can be easily attained, for example, by a stirring treatment using rotary blades.

Specifically, the dissociation treatment can be attained by stirring the particles with a Henschel mixer. The Henschel mixer is known and has a structure having one rotary blade or a plurality of rotary blades in a vessel. The Henschel mixer is usually used for the purpose of stirring and mixing a powder or the like by rotating rotary blades but in the present invention, a shearing action suitable for the purpose of dissociating the aggregated or steric structure of the mixed crystal particle synthesized is provided. The Henschel mixer is preferably constituted by a vessel having a plurality of rotary blades differing in the shape in view of the efficiency, but the rotary blades may have the same shape or one rotary blade may be used. As for the shape of rotary blade, any shape including paddle type, propeller type and turbine type can be used, but a propeller type where the lower blade has a shape of bringing about a powder flow in the entire vessel and the upper blade has a shape of mainly giving shearing suitable for the dissociation of the aggregated or steric structure of the powder is preferred. The powder is charged into the vessel of a Henschel mixer and stirred preferably under the conditions such that the peripheral speed of rotary blade is from 4 to 60 m/s and the stirring time is at least 10 minutes. The blades differing in the shape may be rotated at the same speed or at different speeds. Generally, if the peripheral speed exceeds the above-described range, the powder flies to render ineffectual the shearing action on the powder, failing to destroying the steric structure of the powder, and also the blade is abraded to increase impurities. On the other hand, if the peripheral speed is less than this range, the shearing force is poor and the steric structure cannot be destroyed. If the treatment time is longer, the vessel and rotary blade are more abraded and, therefore, the treatment time must be shortened as much as possible to maintain purity.

In the titania-silica mixed crystal particles produced by the above-described particle synthesis process of the present invention, the dissociation of the aggregated or steric structure is facilitated and, therefore, the method and conditions for the dissociation treatment are not particularly limited. The aggregated or steric structure can be easily dissociated by adding a slight shearing force to the synthesized particles. The production process of titania-silica mixed crystal particles having a high bulk density of the present invention is characterized in that titania-silica mixed crystal particles are produced by such a particle synthesis process ensuring easy dissociation and then subjected to a treatment of dissociating the aggregated or steric structure.

The titania-silica mixed crystal particles produced by the above-described particle synthesis process of the present invention and then subjected to a dissociation treatment are fine primary particles (having a large specific surface area) synthesized by the gas phase process, nevertheless, these are fine or ultrafine particles not having a low bulk density (not grown to aggregated or steric structure) peculiar to the particles synthesized by the gas phase process but having a high bulk density reduced in the aggregation or steric structure.

The titania-silica mixed crystal particles (ultrafine particles) obtained by the production process of the present invention are described in more detail below. The titania-silica mixed crystal particles are a powder obtained by the gas phase process and having a high bulk density of 0.15 to 0.8 $g/cm^3$ despite a high specific surface area of 10 to 200 $m^2/g$. The bulk density is preferably from 0.2 to 0.6 $g/cm^3$ with a specific surface area of 20 to 100 $m^2/g$, more preferably from 0.2 to 0.5 $g/cm^3$ with a specific surface area of 30 to 70 $m^2/g$.

The silica concentration in the particles can be adjusted to from 0.1 mass% to less than 50 mass%, preferably from 10 to 40 mass%, more preferably from 15 to 30 mass%.

Generally, the powder obtained by the gas phase process has a very low bulk density and, therefore, this powder is deficient in that dusting readily occurs on use and the workability at the mixing with other components is bad. However, the powder of the present invention has a high bulk density despite a high specific surface and therefore, is improved not only in such workability but also in the properties described below.

That is, the titania-silica mixed crystal particles of the present invention is characterized by having a low oil absorption amount as an index for the aggregation degree of powder, despite its high specific surface area. As the aggregation degree is higher and the primary particle is smaller, the oil absorption amount is higher. The titania-silica mixed crystal particles obtained by the present invention have an oil absorption amount of less than 1.0 ml/g as measured by using squalane. If the oil absorption amount is high, the powder dispersed or kneaded in cosmetic, compound or the like exhibits a behavior such as gelling or solidification. The powder of the present invention has a small oil absorption amount despite being fine particles and, therefore, a large amount of the powder can be dispersed in a cosmetic, a resin or the like. Moreover, this powder can be applied to these uses without hydrophobing the surface and a formulation fully making use of the characteristics of powder can be obtained.

The oil absorption amount is measured by the method described in JIS K 5101. However, the oil used is squalane in place of the linseed oil described in the JIS.

The method for measuring the oil absorption amount performed in the present invention is described. A sample (5 g) is placed on a glass plate (about 250x250x5 mm), squalane is dropped little by little on the center of sample from a burette and the entire is thoroughly kneaded with a spatula every each dropping. The operation of dropping and kneading is repeated by setting the end point to the time when the entire amount first becomes a solid putty lump to such an extent that the lump can be rolled up into a spiral shape with a steel spatula. The amount of squalane used is determined and the oil absorption amount (ml/g) G is calculated according to the following formula. When, depending on the kind of sample, a putty lump cannot be rolled up into a spiral shape, the time immediately before the lump is abruptly softened by one drop of squalane and sticks on the glass plate is set as the end point.

$$G = H / S$$

wherein

H: amount of squalane (ml)
S: mass of sample (g)
G: oil absorption amount (ml/g).

The bulk density of powder is described below.
The ultrafine particles obtained by the gas phase process are characterized by having a very small bulk density because the characteristic steric structure of primary/secondary particles grows, and this raises a serious problem in the handling of powder. However, the titania-silica mixed crystal particles of the present invention have a large bulk density and therefore, are free from such a problem.

The titania-silica mixed crystal particles of the present invention can be used for almost the same uses of conventional titanium oxide, such as a resin product, a rubber product, a paper, a cosmetic, a paint, a printing ink, a ceramic product, a paste for a dye-sensitized solar cell, and a photo-catalyst. In particular, the titania-silica mixed crystal particles of the present invention can be preferably used for uses where the expression of photocatalytic ability is limited and dispersibility in a medium is required.

The titania-silica mixed crystal particles of the present invention can be used as a composition by adding it to, for example, an organic polymer. Examples of the organic polymer include synthetic thermoplastic resins, synthetic thermosetting resins and natural resins. Specific examples of the organic polymer include polyolefins such as polyethylene, polypropylene and polystyrene, polyamides such as nylon 6, nylon 66 and aramid, polyesters such as polyethylene terephthalate and unsaturated polyester, polyvinyl chloride, polyvinylidene chloride, polyethylene oxide, polyethylene glycol, silicon resin, polyvinyl alcohol, vinyl acetal resin, polyacetate, ABS resin, epoxy resin, vinyl acetate resin, cellulose, cellulose derivatives (e.g., rayon), polyurethane, polycarbonate, urea resin, fluororesin, polyvinylidene fluoride, phenolic resin, celluloid, chitin, starch sheet, acrylic resin, melamine resin and alkyd resin.

The titania-silica mixed crystal particles of the present invention can be used as a composition by adding them to, for example, a silicon polymer.

The organic polymer composition or silicon polymer composition containing the titania-silica mixed crystal particles of the present invention can be used in the form of, for example, a coating material (coating composition), a compound (for example, a resin composition containing the powder) or a molding masterbatch containing the titania-silica mixed crystal particles in a high concentration. In the organic polymer composition or silicon polymer composition, additives such as an antioxidant, an antistatic agent and a metal fatty acid salt may be added.

The concentration of the titania-silica mixed crystal particles of the present invention in the organic polymer composition or silicon polymer composition is preferably from 0.01 to 80 mass%, more preferably from 1 to 50 mass%, based on the total mass of the composition.

By molding such a polymer composition, a molded article having an ultraviolet-shielding ability is obtained. Examples of the molded article include fibers, films and plastic molded articles.

The titania-silica mixed crystal particles of the invention can also be formed into a coating agent by dispersing them in water or an organic solvent and then optionally adding a binder. The binder material is not particularly limited and may be an organic binder or an inorganic binder.

Examples of the binder include polyvinyl alcohol, melamine resin, urethane resin, celluloid, chitin, starch sheet, polyacrylamide, acrylamide, polyester (e.g., unsaturated polyester), polyvinyl chloride, polyvinylidene chloride, polyethylene oxide, polyethylene glycol, silicon resin, vinyl acetal resin, epoxy resin, vinyl acetate resin, polyurethane, urea resin, fluororesin, polyvinylidene fluoride and phenolic resin. Examples of the inorganic binder include zirconium compounds such as zirconium oxychloride, zirconium hydroxychloride, zirconium nitrate, zirconium sulfate, zirconium acetate, ammonium zirconium carbonate and zirconium propionate, silicon compounds such as alkoxysilane and silicate, and metal alkoxides such as aluminum and titanium.

The amount of binder added in the coating agent is preferably from 0.01 to 20 mass%, more preferably from 1 to 10 mass%.

If the binder content is less than 0.01 mass%, a sufficiently high adhesion cannot be obtained after coating, whereas if it exceeds 20 mass%, problems such as increase of viscosity arise and this is also disadvantageous in view of profitability.

The titania-silica mixed crystal particles of the present invention may also be applied to the surface of a structure. The structure is not particularly limited and may be constituted by an inorganic material such as metal, concrete, glass and earthenware, an organic material such as paper, plastic, wood and leather, or a combination thereof. Examples of the structure include building materials, machines, vehicles, glass products, home electric appliances, agricultural materials, electronic equipment, tools, tableware, bath furnishings, toilet goods, furniture, clothing, cloth products, fibers, leather products, paper products, sporting goods, bedding, containers, spectacles, billboards, piping, wiring, metal fittings, hygiene materials, automobile equipment, outdoor products such as tents, stockings, socks, gloves and masks.

The method of applying the titania-silica mixed crystal particle on the surface of a structure is not particularly limited and, for example, the above-described organic polymer composition, silicon polymer composition or coating agent may be directly coated on the structure or may be coated on a structure where a coating film is already provided. Furthermore, another coating film may be further formed thereon.

The titania-silica mixed crystal particles of the present invention can also be used for a cosmetic material or the like. In this cosmetic material, various additives commonly used for cosmetics can be added, such as oil, a whitening agent, a moisturizer, an anti-aging agent, an emollient, essences, an anti-inflammatory, an antioxidant, a surfactant, a chelating agent, an antibiotic, an antiseptic, an amino acid, sugars, an organic acid, alcohols, esters, fats and oils, hydrocarbons, an ultraviolet inhibitor and an inorganic powder.

Specific examples thereof include solvents such as ethanol, isopropanol, butyl alcohol and benzyl alcohol, polyhydric alcohols such as glycerin, propylene glycol, sorbitol, polyethylene glycol, dipropylene glycol, 1,3-butylene glycol and 1,2-pentane diol, sugars such as sorbitol, disaccharides such as trehalose, moisturizers such as hyaluronic acid and water-soluble collagen, vegetable oils such as hydrogenated squalane, olive oil and jojoba oil, emollients such as ceramides, whitening agents such as stable ascorbic acid (e.g., magnesium ascorbic acid phosphate, ascorbic acid glucoside), arbutin, kojic acid, ellagic acid, rucinol and chamomile extract, anti-inflammatory such as allantoin, glycyrrhizinic acid and salts thereof, nonionic surfactants such as glycerin monostearate, POE sorbitan fatty acid ester, sorbitan fatty acid ester, POE alkyl ether, POE•POP block polymer and POE hydrogenated castor oil, anionic surfactants such as fatty acid soap and sodium alkylsulfate, hydrocarbons such as squalane, liquid paraffin, paraffin, isoparaffin, petrolatum and $\alpha$-olefin oligomer, oils and fats such as almond oil, cacao oil, macadamia nut oil, avocado oil, castor oil, sunflower seed oil, evening primrose oil, safflower oil, rape seed oil, horse oil, beef tallow and synthetic triglyceride, waxes such as beeswax, lanolin and jojoba oil, fatty acids such as lauric acid, stearic acid, oleic acid, isostearic acid, myristic acid, palmitic acid, behenic acid, glycolic acid and tartaric acid, higher alcohols such as cetanol, stearyl alcohol, behenyl alcohol and octyl dodecyl alcohol, synthetic esters such as glycerin triester and pentaerythritol tetraester, silicone oils such as dimethylpolysiloxane and methylphenyl-polysiloxane, chelating agents such as EDTA, gluconic acid, phytic acid and sodium polyphosphate, antiseptics and antibiotics such as paraben, sorbic acid, isopropyl-methylphenol, creosol, benzoic acid, ethyl benzoate, stearyldimethylbenzylammonium chloride, hinokitiol, furfural and sodium pyrithione, antioxidants such as vitamin E, dibutylhydroxytoluene, sodium hydrogensulfite and butylhydroxyanisole, buffering agents such as citric acid, sodium citrate, lactic acid and sodium lactate, amino acids such as glycine and alanine, esters such as butyl myristate, ethyl oleate and ethyl stearate, perfumes, pigments, animal and plant extracts, vitamins such as vitamin A, B group and C, vitamin derivatives, ultraviolet absorbents such as paraaminobenzoic acid, octyl paradimethylaminobenzoate, ethyl paraaminobenzoate, phenyl salicylate, benzyl cinnamate, octylmethoxy cinnamate, cinoxate, ethyl urocanate, hydroxymethoxybenzophenone and dihydroxybenzophenone, inorganic powders such as mica, talc, kaolin, calcium carbonate, silicic anhydride, aluminum oxide, magnesium carbonate, barium sulfate, cerium oxide, red oxide of iron, chromium oxide, ultramarine, black iron oxide and yellow iron oxide, and resin powders such as nylon powder and polymethyl methacrylate powder.

The cosmetic material of the present invention can be produced by using a technique commonly employed in the production except for the portion related to the present invention.

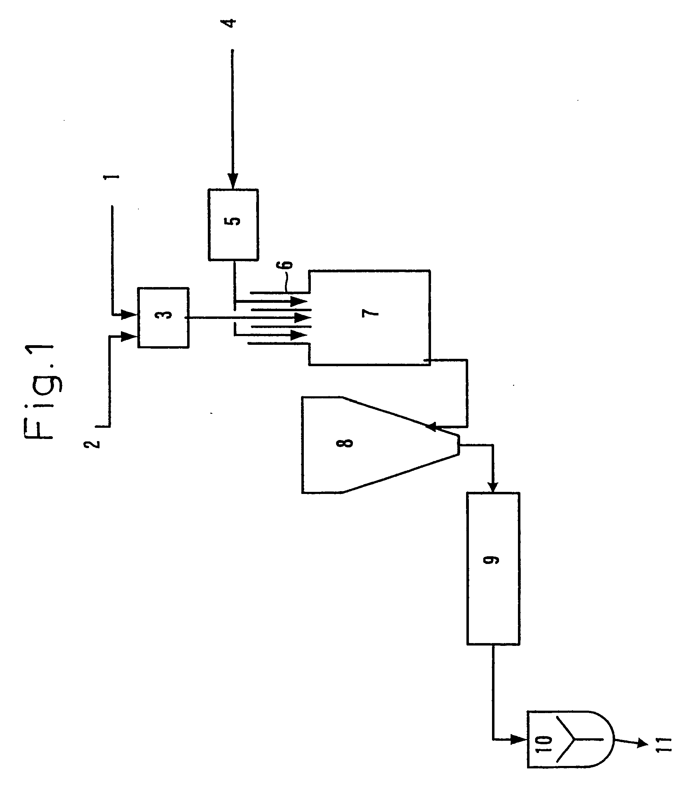

Fig. 1 is a schematic view roughly showing a reaction tube equipped with a coaxial parallel flow nozzle, which is used for the production of the titania-silica mixed crystal particle of the present invention. A titanium halide gas 1 and a silicon halide gas 2 each diluted with an inert gas, if desired, are mixed and preheated to a predetermined temperature by a preheater 3 and then introduced into a reaction tube 7 through the inner tube of a coaxial parallel flow nozzle part 6. An oxidizing gas 4 is preheated to a predetermined temperature by a preheater 5 and then introduced into the reaction tube 7 through the outer tube of the coaxial parallel flow nozzle part 6. The gases introduced into the reaction tube are mixed and reacted and the reactant is rapidly cooled with a cooling gas and then transferred to a bag filter 8 to collect the titania-silica mixed crystal particles. The titania-silica mixed crystal particle collected is transferred to a rotary kiln 9 and heated. The resulting highly purified titania-silica mixed crystal particles are transferred to a volume reducer 10 and reduced in the volume, whereby titania-silica mixed crystal particles 11 having a high bulk density are obtained.

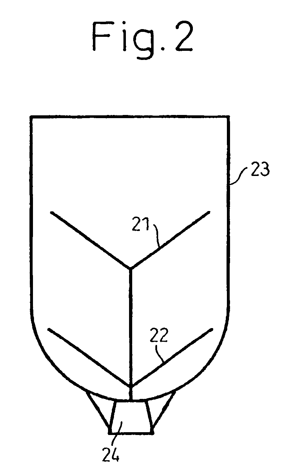

Fig. 2 shows, as an example of the volume reducer 10, a Henschel mixer having two-stage stirring blades 21 and 22 within a vessel 23. The stirring blades 21 and 22 are rotated by a motor 24 and thereby exert the function of stirring and mixing the powder. By the shearing force thereof, the aggregated or steric structure of particles can be dissociated. In view of the particle-stirring action, the stirring blades 21 and 22 are preferably different in the shape.

Examples

The present invention is described in greater detail below by referring to Examples, however, the present invention is not limited to these Examples.

(Evaluation of Composite State)

In the present invention, XPS (X-ray photoelectron spectroscopy) is employed as the method for inspecting the composite state. The details thereof are described, for example, in A. Yu. Stakheev et al., J. Phys. Chem., 97(21), 5668-5672 (1993).

Example 1:

Titanium tetrachloride (40 kg/hr), silicon tetrachloride (15 kg/hr) and nitrogen gas (5 $Nm^3$/hr) were introduced into a vaporizer and the obtained gas was heated to 1,000°C. This is called a raw material gas. Separately, a mixed gas of oxygen (5 $Nm^3$/hr) and water vapor (30 $Nm^3$/hr) was heated similarly to 1,000°C. This is called an oxidizing gas. The thus-obtained two gases (raw material gas and oxidizing gas) were introduced into a reaction tube at 70 m/s (raw material gas) and 110 m/s (oxidizing gas) through a coaxial parallel flow nozzle. The high temperature residence time was calculated to be 0.78 seconds. The powder obtained by this reaction was collected with a bag filter and then introduced into an external heating rotary kiln heated at 800°C to remove the adsorbed by-product component (hydrogen chloride). The residence time in the rotary kiln was about 30 minutes.

The thus-obtained powder (1 kg) was charged into a Henschel mixer manufactured by Mitsui Mining Co., Ltd. (Model: FM10B, upper blade: ST type, lower blade: A0 type) and stirred at a peripheral speed of 40 m/s for 20 minutes.

The powder obtained was white and the specific surface area thereof was measured using a Monosorb-type apparatus manufactured by Quantachrome Corp. according to the BET one-point method and found to be 54 $m^2$/g. The powder was analyzed using a fluorescent X-ray analyzer, X-ray Spectrometer Simultix 10, manufactured by Rigaku Corporation and found to contain 21 mass% of a silica component. The bulk density was 0.30 $g/cm^3$, the HCl content was 0.6 mass% and the oil absorption amount was 0.86 ml/g. Furthermore, by the local component analysis (EDX), this titania-silica mixed crystal was confirmed to be a titania-silica mixed crystal particle where the silica component (fine crystal) was uniformly dispersed in the titanium oxide fine crystal.

Example 2:

Titanium tetrachloride (15 kg/hr), silicon tetrachloride (12 kg/hr) and nitrogen gas (30 $Nm^3$/hr) were introduced into a vaporizer and the obtained gas was heated to 1,100°C. This is called a raw material gas. Separately, a mixed gas of oxygen (5 $Nm^3$/hr) and water vapor (42 $Nm^3$/hr) was heated similarly to 1,100°C. This is called an oxidizing gas. The thus-obtained two gases (raw material gas and oxidizing gas) were introduced into a reaction tube at 74 m/s (raw material gas) and 125 m/s (oxidizing gas) through a coaxial parallel flow nozzle. The high temperature residence time was calculatively 0.51 seconds. The powder obtained by this reaction was collected with a bag filter and then introduced into an external heating rotary kiln heated at 800°C to remove the by-product component (hydrogen chloride) adsorbed. The residence time in the rotary kiln was about 60 minutes.

The thus-obtained powder (1 kg) was charged into a Henschel mixer manufactured by Mitsui Mining Co., Ltd. (Model: FM10B, upper blade: ST type, lower blade: A0 type) and stirred at a peripheral speed of 23 m/s for 20 minutes.

The powder obtained was white and the specific surface area thereof was measured using a Monosorb-type apparatus manufactured by Quantachrome Corp. according to the BET one-point method and found to be 92 $m^2$/g. The powder was analyzed using a fluorescent X-ray analyzer, X-ray Spectrometer Simultix 10, manufactured by Rigaku Corporation and found to contain 38 mass% of a silica component. The bulk density was 0.2 $g/cm^3$, the HCl content was 0.8 mass% and the oil absorption amount was 0.93 ml/g. Furthermore, by the local component analysis (EDX), this titania-silica mixed crystal was confirmed to be titania-silica mixed crystal particles where the silica component was uniformly dispersed in the titanium oxide fine crystal.

Example 3:

A foundation having the following formulation was produced by a normal method. As the titania-silica mixed crystal powder, the titania-silica mixed crystal particle obtained in Example 1 was used:

Formulation of Foundation:

| | |
|---|---|
| Titania-silica mixed crystal particle | 30 mass% |
| Mica | 15 mass% |
| Talc | 10 mass% |
| Iron oxide (red) | 1.5 mass% |
| Iron oxide (yellow) | 3.5 mass% |
| Glycerin | 10 mass% |
| Purified water | 30 mass% |
| Perfume | optimum |

This foundation gave clearness and a good feeling on use.

Comparative Example 1:

Titanium tetrachloride (40 kg/hr), silicon tetrachloride (15 kg/hr) and nitrogen gas (5 $Nm^3$/hr) were introduced into a vaporizer and the obtained gas was heated to 1,000°C. This is called a raw material gas. Separately, a mixed gas of oxygen (5 $Nm^3$/hr) and water vapor (30 $Nm^3$/hr) was heated similarly to 1,000°C. This is called an oxidizing gas. The thus-obtained two gases (raw material gas and oxidizing gas) were introduced into a reaction tube at 70 m/s (raw material gas) and 110 m/s (oxidizing gas) through a coaxial parallel flow nozzle. The high temperature residence time was calculated to be 0.78 seconds. The powder obtained by this reaction was collected with a bag filter and then introduced into an external heating rotary kiln heated at 800°C to remove the by-product component (hydrogen chloride) adsorbed. The residence time in the rotary kiln was about 30 minutes.

The powder obtained was white and the specific surface area thereof was measured using a Monosorb-type apparatus manufactured by Quantachrome Corp. according to the BET one-point method and found to be 54 $m^2$/g. The powder was analyzed using a fluorescent X-ray analyzer, X-ray Spectrometer Simultix 10, manufactured by Rigaku Corporation and found to contain 21 mass% of silica component. The bulk density was 0.12 $g/cm^3$, the HCl content was 0.6 mass% and the oil absorption amount was 1.1 ml/g. Furthermore, by the local component analysis (EDX), this titania-silica mixed crystal was confirmed to be a titania-silica mixed crystal particle where the silica fine crystal was uniformly dispersed in the titanium oxide fine crystal.

Comparative Example 3:

A foundation having the following formulation was produced by a normal method. As the titania-silica mixed crystal powder, the titania-silica mixed crystal particle obtained in Comparative Example 1 was used:

Formulation of Foundation:

| | |
|---|---|
| Titania-silica mixed crystal particle | 30 mass% |
| Mica | 15 mass% |
| Talc | 10 mass% |
| Iron oxide (red) | 1.5 mass% |
| Iron oxide (yellow) | 3.5 mass% |
| Glycerin | 10 mass% |
| Purified water | 30 mass% |
| Perfume | optimum |

This foundation was solidified during blending and a uniform foundation could not be obtained.

Industrial Applicability

The titania-silica mixed crystal particles of the present invention is excellent in the visible light-transmitting property and also in the ultraviolet-shielding ability and readily dispersible in a medium and therefore, the particles can be preferably used particularly for a composition required to have transparency and ultraviolet-shielding property. Furthermore, the titania-silica mixed crystal particles of the present invention are partially restrained in surface activity and do not decompose an organic composition present together and therefore, this particle can be used without applying a surface treatment. In addition, the titania-silica mixed crystal particles can exert a photocatalytic ability.

**Claims**

1. A process for producing titania-silica mixed crystal particles having a high bulk density and comprising titanium oxide as the main component and silicon oxide as a subsidiary component, the process comprising decomposing gaseous titanium halide and gaseous silicon halide each heated at 600°C or more in the presence of oxygen or water vapor heated at 600°C or more, the residence time of the gaseous titanium halide and gaseous silicon halide at a high temperature of 600°C or more being 1 second or less, to obtain a powder comprising titanium oxide and silicon oxide, heating the obtained powder at 500 to 800°C to decrease the concentration of raw material-originated hydrogen halide in the powder to 1 mass% or less, and then subjecting the powder to a treatment of dissociating the aggregated or steric structure.

2. The process as described in claim 1, wherein in the step of said decomposition, the gaseous metal halide and an oxidizing gas are introduced into a reactor at a flow rate of 30 m/sec or more.

3. The process as described in claim 2, wherein the gaseous metal halide and the oxidizing gas have an average flow rate of 5 m/sec or more in the reactor.

4. The process as described in claim 2, wherein the gaseous metal halide and the oxidizing gas have a residence time at a temperature of 600°C or more in the reactor is 1 second or less.

5. The process for producing a titania-silica mixed crystal particle having a high bulk density as described in any one of claims 1 to 4, wherein the treatment of dissociating the aggregated or steric structure is a stirring treatment of charging the powder into a vessel having a plurality of rotary blades differing in the shape and rotating the rotary blades at a peripheral speed of 4 to 60 m/s.

6. The process according to claim 5, wherein the dissociating treatment of the powder is conducted by a Henschel mixer.

7. Titania-silica mixed crystal particles obtainable by the production process described in any one of claims 1 to 6, which has a BET specific surface area of 10 to 200 $m^2/g$ and a bulk density of 0.15 $g/cm^3$ to less than 0.8 $g/cm^3$.

8. Titania-silica mixed crystal particles according to claim 7, which have a BET specific surface area of 20 to 100 $m^2/g$ and a bulk density of 0.2 $g/cm^3$ to less than 0.6 $g/cm^3$.

9. Titania-silica mixed crystal particles according to claim 7 or 8, which have a BET specific surface area of 30 to 70 $m^2/g$ and a bulk density or 0.2 $g/cm^3$ to less than 0.5 $g/cm^3$.

10. The titania-silica mixed crystal particles as described in any one of claims 7 to 9, wherein $SiO_2$ is contained in an amount of 0.1 mass% to less than 50 mass%.

11. The titania-silica mixed crystal particles according to claim 10, wherein $SiO_2$ is contained in an amount of 10 to 40 mass%.

12. The titania-silica mixed crystal particles according to claim 10, wherein $SiO_2$ is contained in an amount of 15 to 30 mass%.

13. The titania-silica mixed crystal particles as described in any one of claims 7 to 12, wherein the oil absorption amount is less than 1 ml/g as measured by the oil absorption measuring method of JIS K 5101 using squalane in place of linseed oil.

14. A cosmetic material comprising the titania-silica mixed crystal particles described in any one of claims 7 to 13.

15. The cosmetic material as described in claim 14, further comprising an additive selected from the group consisting of oils, whitening agents, moisturizers, anti-aging agents, emollients, essences, antiinflammatories, antioxidants, surfactants, chelating agents, antibiotics, antiseptics, amino acids, sugars, organic acids, alcohols, esters, fats and oils, hydrocarbons, ultraviolet inhibitors and inorganic powders.

16. An organic polymer composition comprising an organic polymer and the titania-silica mixed crystal particles described in any one of claim 7 to 13, the titania-silica mixed crystal particles being contained in an amount of 0.01 to 80 mass% based on the total mass of the composition.

17. The organic polymer composition as described in claim 16, wherein the organic polymer of the organic polymer composition is at least one resin selected from the group consisting of synthetic thermoplastic resins, synthetic thermosetting resins and natural resins.

18. A silicon polymer composition comprising a silicon polymer and the titania-silica mixed crystal particles described in any one of claims 7 to 13, the titania-silica mixed crystal particles being contained in an amount of 0.01 to 90 mass% based on the total mass of the composition.

19. The organic polymer composition or silicon polymer composition as described in any one of claims 15 to 18, wherein the organic polymer composition or silicon polymer composition is a compound.

20. The organic polymer composition or silicon polymer composition as described in any one of claims 15 to 18, wherein the organic polymer composition or silicon polymer composition is a masterbatch.

21. A molded article obtained by molding the organic polymer composition or silicon polymer composition described in any one of claims 15 to 20.

22. The molded article as described in claim 21, wherein the molded article is one selected from the group of fiber, film and plastic molded article.

**23.** A slurry comprising the titania-silica mixed crystal particle described in any one of claims 7 to 13.

**24.** A dye-sensitized solar cell comprising the titania-silica mixed crystal particles described in any one of claims 7 to 13 in the structure.

**25.** A coating agent comprising the titania-silica mixed crystal particles described in any one of claims 7 to 13 in water or an organic solvent and optionally a binder.

**26.** A coating material comprising the titania-silica mixed crystal particle described in any one of claims 7 to 13 in water or an organic solvent and optionally a binder.

**27.** A structure having on the surface thereof the titania-silica mixed crystal particle described in any one of claims 7 to 13.

**28.** The structure as described in claim 27, which is selected from the group consisting of building materials, machines, vehicles, glass products, home electric appliances, agricultural materials, electronic equipment, tools, tableware, bath furnishings, toilet goods, furniture, clothing, cloth products, fibers, leather products, paper products, sporting goods, bedding, containers, spectacles, billboards, piping, wiring, metal fittings, hygiene materials, automobile equipment, outdoor products such as tents, stockings, socks, gloves and masks.

**29.** A photocatalyst which is the titania-silica mixed crystal particle described in any one of claims 7 to 13.


**Patentansprüche**

**1.** Verfahren zur Herstellung von Titandioxid-Siliciumdioxid-Mischkristallteilchen mit einer hohen Schüttdichte, umfassend Titanoxid als Hauptkomponente und Siliciumoxid als Nebenkomponente, wobei das Verfahren das Zersetzen von jeweils auf 600°C oder höher erhitztem gasförmigem Titanhalogenid und gasförmigem Siliciumhalogenid, die jeweils in Gegenwart von auf 600°C oder höher erhitztem Sauerstoff oder Wasserdampf erhitzt wurden, wobei die Verweilzeit des gasförmigen Titanhalogenids und gasförmigen Siliciumhalogenids bei einer hohen Temperatur von 600°C oder mehr 1 Sekunde oder weniger ist, um ein Pulver zu erhalten, welches Titanoxid und Siliciumoxid enthält,
das Erhitzen des erhaltenen Pulvers bei 500 bis 800°C, um die Konzentration des aus dem Rohmaterial stammenden Wasserstoffhalogenids in dem Pulver auf 1 Massen-% oder weniger zu vermindern, und

anschließend das Unterziehen des Pulvers einer Behandlung zum Dissoziieren der aggregierten oder sterischen Struktur beinhaltet.

**2.** Verfahren nach Anspruch 1, worin in dem Zersetzungsschritt das gasförmige Metallhalogenid und ein oxidierendes Gas bei einer Strömungsgeschwindigkeit von 30 m/s oder mehr in einen Reaktor eingeführt werden.

**3.** Verfahren nach Anspruch 2, worin das gasförmige Metallhalogenid und das oxidierende Gas eine durchschnittliche Strömungsgeschwindigkeit von 5 m/sec oder mehr in dem Reaktor aufweisen.

**4.** Verfahren nach Anspruch 2, worin das gasförmige Metallhalogenid und das oxidierende Gas eine Verweilzeit bei einer Temperatur von 600°C oder mehr in dem Reaktor von 1 Sekunde oder weniger aufweisen.

**5.** Verfahren zur Herstellung eines Titandioxid-Siliciumdioxid-Mischkristallteilchens mit hoher Schüttdichte nach einem der Ansprüche 1 bis 4, worin die Behandlung zum Dissoziieren der aggregierten oder sterischen Struktur eine Rührbehandlung ist, bei der das Pulver in ein Gefäß mit einer Mehrzahl von Rührblättern, die sich in der Gestalt unterscheiden, eingeführt wird, und die Rührblätter mit einer Umfangsgeschwindigkeit von 4 bis 60 m/s rotiert werden.

**6.** Verfahren nach Anspruch 5, worin die Dissoziierungsbehandlung des Pulvers mittels eines Henschel-Mischers durchgeführt wird.

**7.** Titandioxid-Siliciumdioxid-Mischkristallteilchen, erhältlich durch das in einem der Ansprüche 1 bis 6 beschriebene Herstellungsverfahren, wobei diese einen spezifischen Oberflächenbereich nach BET von 10 bis 200 $m^2$/g und eine Schüttdichte von 0,15 $g/cm^3$ bis weniger als 0,8 $g/cm^3$ aufweisen.

**8.** Titandioxid- Siliciumdioxid- Mischkristallteilchen nach Anspruch 7, wobei diese einen spezifischen Oberflächenbereich nach BET von 20 bis 100 $m^2$/g und eine Schüttdichte von 0,2 $g/cm^3$ bis weniger als 0,6 $g/cm^3$ aufweisen.

**9.** Titandioxid- Siliciumdioxid- Mischkristallteilchen nach Anspruch 7, wobei diese einen spezifischen Oberflächenbereich nach BET von 30 bis 70 $m^2$/g und eine Schüttdichte von 0,2 $g/cm^3$ bis weniger als 0,65 $g/cm^3$ aufweisen.

**10.** Titandioxid- Siliciumdioxid- Mischkristallteilchen nach einem der Ansprüche 7 bis 9, worin $SiO_2$ in einer Menge von 0,1 Massen-% bis weniger als 50 Massen-% enthalten ist.

11. Titandioxid- Siliciumdioxid- Mischkristallteilchen nach Anspruch 10, worin $SiO_2$ in einer Menge von 10 bis 40 Massen-% enthalten ist.

12. Titandioxid- Siliciumdioxid- Mischkristallteilchen nach Anspruch 10, worin $SiO_2$ in einer Menge von 15 bis 30 Massen-% enthalten ist.

13. Titandioxid- Siliciumdioxid- Mischkristallteilchen nach einem der Ansprüche 7 bis 12, wobei die Ölabsorptionsmenge jeweils 1 ml/g beträgt, gemessen mittels des Ölabsorptionsmessverfahrens von JIS K 5101 unter Verwendung von Squalan anstelle von Leinsamenöl.

14. Kosmetisches Material, welches die Titandioxid-Siliciumdioxid-Mischkristallteilchen nach einem der Ansprüche 7 bis 13 enthält.

15. Kosmetisches Material nach Anspruch 14, welches weiterhin ein Additiv enthält, ausgewählt aus der Gruppe, bestehend aus Ölen, Weißmachmitteln, Feuchthaltemitteln, Antialterungsmitteln, Erweichungsmitteln (Emollients), Essenzen, Antientzündungsmitteln, Antioxidantien, oberflächenaktiven Mitteln, Komplexierungsmitteln, Antibiotika, Antiseptika, Aminosäuren, Zuckern, organischen Säuren, Alkoholen, Estern, Fetten und Ölen, Kohlenwasserstoffen, Ultraviolettinhibitoren und anorganischen Pulvern.

16. Organische Polymerzusammensetzung, welche ein organisches Polymer und die Titandioxid-Siliciumoxid-Mischkristallteilchen nach einem der Ansprüche 7 bis 13 enthalten und wobei die Titandioxid-Siliciumdioxid-Mischkristallteilchen in einer Menge von 0,01 bis 80 Massen-% enthalten sind, bezogen auf die gesamte Masse der Zusammensetzung.

17. Organische Polymerzusammensetzung nach Anspruch 16, worin das organische Polymer der organischen Polymerzusammensetzung mindestens ein Harz ist, ausgewählt aus der Gruppe, bestehend aus synthetischen thermoplastischen Harzen, synthetischen wärmehärtbaren Harzen und natürlichen Harzen.

18. Siliconpolymerzusammensetzung, umfassend ein Siliconpolymer und die Titandioxid-Siliciumdioxid-Mischkristallteilchen nach einem der Ansprüche 7 bis 13, wobei die Titandioxid-Siliciumdioxid-Mischkristallteilchen in einer Menge von 0,01 bis 90 Massen-% enthalten sind, bezogen auf die gesamte Masse der Zusammensetzung.

19. Organische Polymerzusammensetzung oder Siliconpolymerzusammensetzung nach einem der Ansprüche 15 bis 18, worin die organische Polymerzusammensetzung oder Siliconpolymerzusammensetzung ein Gemisch ist.

20. Organische Polymerzusammensetzung oder Siliconpolymerzusammensetzung nach einem der Ansprüche 15 bis 18, worin die organische Polymerzusammensetzung oder Siliconpolymerzusammensetzung ein Masterbatch ist.

21. Formgegenstand, erhalten durch Formen der organischen Polymerzusammensetzung oder Siliconpolymerzusammensetzung nach einem der Ansprüche 15 bis 20.

22. Formgegenstand nach Anspruch 21, worin der Formgegenstand aus der Gruppe Fasern, Folien und Kunststoffformgegenstand ausgewählt ist.

23. Aufschlämmung, welche das Titandioxid-Siliciumdioxid-Mischkristallteilchen nach einem der Ansprüche 7 bis 13 enthält.

24. Farbsensibilisierte Solarzelle, welche die Titandioxid-Siliciumdioxid-Mischkristallteilchen nach einem der Ansprüche 7 bis 13 in ihrer Struktur enthält.

25. Beschichtungsmittel, welches die Titandioxid-Siliciumdioxid-Mischkristallteilchen nach einem der Ansprüche 7 bis 13 in Wasser oder einem organischen Lösungsmittel und wahlweise einem Bindemittel enthält.

26. Beschichtungsmaterial, welches die Titandioxid-Siliciumdioxid-Mischkristallteilchen nach einem der Ansprüche 7 bis 13 in Wasser oder einem organischen Lösungsmittel und wahlweise einem Bindemittel enthält.

27. Struktur mit den Titandioxid-Siliciumdioxid-Mischkristallteilchen nach einem der Ansprüche 7 bis 13 auf der Oberfläche.

28. Struktur nach Anspruch 27, wobei diese aus der Gruppe ausgewählt ist, bestehend aus Baumaterialien, Maschinen, Fahrzeugen, Glasprodukten, Heimelektrikanwendungen, landwirtschaftlichen Materialien, elektronischen Gerätschaften, Werkzeugen, Geschirr, Badeinrichtungen, Toilettenwaren, Möbeln, Kleidung, Stoffprodukten, Fasern, Lederprodukten, Papierprodukten, Sportgegenständen, Bettwaren, Behältern, Brillen, Anzeigetafeln, Leitungen, Verdrahtungen, Metallanschlüssen, Hygienematerialien, Automobilgerätschaften, Outdoor-Produkten, wie Zelten, Strümpfen, Socken, Handschuhen und Masken.

29. Fotokatalysator, wobei es sich um das Titandioxid-Siliciumdioxid-Mischkristallteilchen nach einem der Ansprüche 7 bis 13 handelt.

**Revendications**

1. Procédé de production de particules de cristaux mixtes d'oxyde de titane-silice ayant une masse volumique apparente élevée et comprenant de l'oxyde de titane comme constituant principal et de l'oxyde de silicium comme constituant subsidiaire, le procédé consistant à décomposer l'halogénure de titane gazeux et l'halogénure de silicium gazeux chacun chauffé à 600°C ou supérieur en présence d'oxygène ou de vapeur d'eau chauffée à 600°C ou supérieur, le temps de séjour de l'halogénure de titane gazeux et de l'halogénure de silicium gazeux à une température élevée de 600°C ou supérieur étant de 1 s ou inférieur, pour obtenir une poudre comprenant de l'oxyde de titane et de l'oxyde de silicium, à chauffer la poudre obtenue à de 500 à 800°C pour abaisser la concentration de l'halogénure d'hydrogène à l'origine de la matière brute dans la poudre à 1 % en masse ou inférieur et à soumettre ensuite la poudre à un traitement de dissociation de la structure agrégée ou stérique.

2. Procédé selon la revendication 1, dans lequel dans l'étape de ladite décomposition, l'halogénure de métal gazeux et un gaz oxydant sont introduits dans un réacteur à un débit de 30 m/s ou supérieur.

3. Procédé selon la revendication 2, dans lequel l'halogénure de métal gazeux et le gaz oxydant présentent un débit moyen de 5 m/s ou supérieur dans le réacteur.

4. Procédé selon la revendication 2, dans lequel l'halogénure de métal gazeux et le gaz oxydant présentent un temps de séjour à une température de 600°C ou supérieur dans le réacteur de 1 s ou inférieur.

5. Procédé de production de particules de cristaux mixtes d'oxyde de titane-silice ayant une masse volumique apparente élevée comme décrit dans l'une quelconque des revendications 1 à 4, dans lequel le traitement de dissociation de la structure agrégée ou stérique est un traitement d'agitation consistant à charger la poudre dans une cuve présentant plusieurs pales rotatives de forme différente et à faire tourner les pales rotatives à une vitesse périphérique de 4 à 60 m/s.

6. Procédé selon la revendication 5, dans lequel le traitement de dissociation de la poudre est réalisé par un mélangeur Henschel.

7. Particules de cristaux mixtes d'oxyde de titane-silice pouvant être obtenues par le procédé de production décrit dans l'une quelconque des revendications 1 à 6, lesquelles présentent une surface spécifique BET de 10 à 200 m$^2$/g et une masse volumique apparente de 0,15 g/cm$^3$ à moins de 0,8 g/cm$^3$

8. Particules de cristaux mixtes d'oxyde de titane-silice selon la revendication 7, lesquelles présentent une surface spécifique BET de 20 à 100 m$^2$/g et une masse volumique apparente de 0,2 g/cm$^3$ à moins de 0,6 g/cm$^3$

9. Particules de cristaux mixtes d'oxyde de titane-silice selon la revendication 7 ou 8, lesquelles présentent une surface spécifique BET de 30 à 70 m$^2$/g et une masse volumique apparente de 0,2 g/cm$^3$ à moins de 0,5 g/cm$^3$.

10. Particules de cristaux mixtes d'oxyde de titane-silice selon l'une quelconque des revendications 7 à 9, dans lesquelles SiO$_2$ est contenu dans une quantité de 0,1 % en masse à moins de 50 % en masse.

11. Particules de cristaux mixtes d'oxyde de titane-silice selon la revendication 10, dans lesquelles SiO$_2$ est contenu dans une quantité de 10 à 40 % en masse.

12. Particules de cristaux mixtes d'oxyde de titane-silice selon la revendication 10, dans lesquelles SiO$_2$ est contenu dans une quantité de 15 à 30 % en masse.

13. Particules de cristaux mixtes d'oxyde de titane-silice selon l'une quelconque des revendications 7 à 12, dans lesquelles la quantité d'absorption d'huile est inférieure à 1 ml/g comme mesuré par le procédé de mesure d'absorption d'huile de JIS K 5101 utilisant du squalane à la place de l'huile de lin.

14. Matériau cosmétique comprenant les particules de cristaux mixtes d'oxyde de titane-silice décrites dans l'une quelconque des revendications 7 à 13.

15. Matériau cosmétique selon la revendication 14 comprenant de plus un additif choisi parmi des huiles, des agents blanchissants, des agents hydratants, des agents anti-âge, des émollients, des essences, des anti-inflammatoires, des antioxydants, des tensioactifs, des agents chélatants, des antibiotiques, des antiseptiques, des aminoacides, des sucres, des acides organiques, des alcools, des esters, des graisses et des huiles, des hydrocarbures, des inhibiteurs d'ultraviolets et des poudres inorganiques.

16. Composition polymère organique comprenant un polymère organique et les particules de cristaux mixtes d'oxyde de titane-silice décrites dans l'une quelconque des revendications 7 à 13, les particules de cristaux mixtes d'oxyde de titane-silice étant contenues dans une quantité de 0,01 à 80 % en masse rapporté à la masse totale de la composition.

17. Composition polymère organique selon la revendication 16, dans laquelle le polymère organique de la composition polymère organique est au moins une résine choisie parmi des résines thermoplastiques synthétiques, des résines thermodurcissables synthétiques et des résines naturelles.

18. Composition polymère de silicium comprenant un polymère de silicium et les particules de cristaux mixtes d'oxyde de titane-silice décrites dans l'une quelconque des revendications 7 à 13, les particules de cristaux mixtes d'oxyde de titane-silice étant contenues dans une quantité de 0,01 à 90 % en masse rapporté à la masse totale de la composition.

19. Composition polymère organique ou composition polymère de silicium selon l'une quelconque des revendications 15 à 18, dans laquelle la composition polymère organique ou la composition polymère de silicium est un composé.

20. Composition polymère organique ou composition polymère de silicium selon l'une quelconque des revendications 15 à 18, dans laquelle la composition polymère organique ou la composition polymère de silicium est un lot-mère.

21. Article moulé obtenu par moulage de la composition polymère organique ou de la composition polymère de silicium décrite dans l'une quelconque des revendications 15 à 20.

22. Article moulé selon la revendication 21, dans lequel l'article moulé est un article choisi parmi une fibre, un film et un article moulé en plastique.

23. Suspension comprenant les particules de cristaux mixtes d'oxyde de titane-silice décrites dans l'une quelconque des revendications 7 à 13.

24. Cellule solaire colorant-sensibilisée comprenant les particules de cristaux mixtes d'oxyde de titane-silice décrites dans l'une quelconque des revendications 7 à 13 dans la structure.

25. Agent de revêtement comprenant les particules de cristaux mixtes d'oxyde de titane-silice décrites dans l'une quelconque des revendications 7 à 13 dans de l'eau ou dans un solvant organique et facultativement un liant.

26. Matériau de revêtement comprenant les particules de cristaux mixtes d'oxyde de titane-silice décrites dans l'une quelconque des revendications 7 à 13 dans de l'eau ou dans un solvant organique et facultativement un liant.

27. Structure présentant sur sa surface les particules de cristaux mixtes d'oxyde de titane-silice décrites dans l'une quelconque des revendications 7 à 13.

28. Structure selon la revendication 27, laquelle est choisie parmi des matériaux de construction, des machines, des véhicules, des produits en verre, des appareils électriques ménagers, des matériaux pour l'agriculture, un équipement électronique, des outils, des objets de table, des meubles de salle de bains, des articles pour la toilette, du mobilier, des vêtements, des produits de tissu, des fibres, des produits en cuir, des produits en papier, des équipements sportifs, de la literie, des récipients, des lunettes, des panneaux d'affichage, des tuyauteries, des câblages, des raccords métalliques de tuyauteries, des matériaux pour l'hygiène, un équipement automobile, des produits pour l'extérieur, tels que des tentes, des collants, des chaussettes, des gants et des masques.

29. Photocatalyseur qui est la particule de cristal mixte d'oxyde de titane-silice décrite dans l'une quelconque des revendications 7 à 13.

# Fig.1

EP 1 587 758 B1

# Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 50115190 A
- JP 2503370 B
- EP 595078 A
- JP 9511985 T
- GB 689123 A
- US 3219468 A
- JP 2001139331 A
- JP 10194741 A

**Non-patent literature cited in the description**

- **A. YU. STAKHEEV et al.** *J. Phys. Chem.,* 1993, vol. 97 (21), 5668-5672